# EUROPÄISCHE PATENTANMELDUNG

(11) **EP 0 695 550 A2**
(43) Veröffentlichungstag der Anmeldung: **07.02.1996**
(21) Anmeldenummer: 95108293.2
(22) Anmeldetag: 31.05.1995
(51) Int. Cl.: A61K 35/78

(54) **Mittel zur Stärkung des menschlichen, insbesondere des männlichen Organismus**

(30) Priorität: 05.08.1994 DE 4427756
(71) Anmelder: Aslan Arzneimittel GmbH, D-44263 Dortmund (DE)
(72) Erfinder: Müller, Rolf Cornelius, Dr., D-59939 Olsberg (DE)
(74) Vertreter: Patentanwälte Meinke, Dabringhaus und Partner

(57) **Zusammenfassung**

Ein Mittel zur Stärkung des menschlichen, insbesondere des männlichen, Organismus soll vorgeschlagen werden, das einem Leistungsabfall, Erschöpfungs- und Schwächezuständen entgegenwirkt und dabei vor allem den Anforderungen eines Nahrungsergänzungsmittels, Diätetikums oder Arzneimittels, insbesondere im Hinblick auf Darreichungsform und Haltbarkeit genügt.

Dies wird durch einen Gehalt an Pastinak-Bestandteilen, Vitaminen und wenigstens einem Stabilisator erreicht.

## Beschreibung

Die Erfindung betrifft ein Mittel zur Stärkung des menschlichen, insbesondere des männlichen Organismus.

Derartige Mittel zur Stärkung und Kräftigung des menschlichen Organismus, insbesondere des männlichen Organismus, mit Libido- und Potenz-steigernden Eigenschaften sind in vielfältigen Ausführungsformen bekannt. Diese Mittel enthalten häufig pflanzliche Naturstoffe, denen entsprechend stärkende Wirkung zuerkannt wird.

Schon seit Zeiten der Antike ist es bekannt gewesen, die Naturpflanze Pastinak zu derartigen Zwecken einzusetzen. Pastinak ist ein beispielsweise in Deutschland heimisches 2-jähriges Doldengewächs. Die offizielle botanische Bezeichnung ist Pastinaca Sativa L (Pahlow, M.: Das große Buch der Heilpflanzen, überarbeitete Neuausgabe, 1. Auflage, München: Gräfe und Unzer, 1993) aus der Familie der Apiaceae (Umbelliferae). Gebräuchliche Volksnamen sind Dickmöhre, Hammelmöhre, Hirschfraß, Pastornak und Spindelwurz. Ernährungsphysiolgisch gesehen zeichnet sich Pastinak durch einen hohen Gehalt an wichtigen Mineralstoffen, aufbauenden Vitaminen und Provitaminen sowie durch eine Vielzahl essentieller Aminosäuren aus, die wiederum im menschlichen Organismus zu physiologisch hochaktiven Neurotransmittern metabolisiert werden (vgl. Senser, F. und Scherz, H.: "Souci-Fachmann-Kraut", Lebensmitteltabelle für die Praxis, Herausgeber Deutsche Forschungsanstalt für Lebensmittelchemie, Garching bei München, 2. überarbeitete und erweiterte Auflage; Wissenschaftliche Verlagsgesellschaft mbH, Stuttgart 1991). Pharmakologisch wirksame Bestandteile des Pastinaks sind anregende ätherische Öle und stoffwechselaktivierende organische Säuren, kräftigende Pflanzenstärke und Pastinacin, ein spezieller nur bei dieser Pflanze auftretender Wirkstoff, der u.a. die Durchblutung fördert (Richter, H.J. und Böhm, M.: Pharmazeutisch-Medizinisches Lexikon; Band 2 L-Z, 1. Auflage, VEB Verlag Volk und Gesundheit Berlin 1989).

Neben der Erwähnung des Pastinaks bereits in der Antike durch beispielsweise Dioskorides und Plinius wurde der Pastinak im Capitulare de villis und im 12. Jahrhundert von Hildegard von Bingen aufgeführt. Schon damals wurde er auch als Arzneipflanze genutzt. Die Kräuterbücher des 16. und 17. Jahrhunderts empfahlen die Wurzel als stärkendes Hausmittel (Ennet, D.: BI-Lexikon "Heilpflanzen und Drogen"; VEB Bibliographisches Institut Leipzig; 1. Auflage 1988). Darüber hinaus hat die Anwendung des Pastinaks als Aphrodisiakum eine lange Tradition in der Volks- und Naturheilkunde.

Trotz dieser langen Bekanntheit und Verwendung des Pastinaks als Stärkungsmittel ist es bis heute nicht gelungen, Stärkungsmittel auf Pastinak-Basis vorzuschlagen, die den heutigen Anforderungen, insbesondere im Hinblick auf Darreichungsform, Haltbarkeit und dergl. gerecht werden.

Aufgabe der Erfindung ist es deshalb, ein Mittel zur Stärkung des menschlichen, insbesondere des männlichen Organismus vorzuschlagen, das einem Leistungsabfall, Erschöpfungs- und Schwächezuständen entgegenwirkt und dabei vor allem den Anforderungen eines Nahrungsergänzungsmittels, Diätetikums oder Arzneimittels, insbesondere im Hinblick auf Darreichungsform und Haltbarkeit genügt.

Diese Aufgabe wird mit einem Mittel zur Stärkung des menschlichen, insbesondere des männlichen Organismus gelöst, das sich durch einen Gehalt an Pastinak-Bestandteilen, Vitaminen und wenigstens einem Stabilisator auszeichnet.

Es hat sich überraschend herausgestellt, daß ein derartiges Mittel auf Pastinak-Basis hervorragend zur Stärkung und Kräftigung des menschlichen, insbesondere des männlichen Organismus geeignet ist, wobei dieses Mittel in Kapsel- bzw. Tablettenform oder auch als Tonikum durch den Zusatz der weiteren Bestandteile (Vitamine und Stabilisator) den Anforderungen an ein Nahrungsergänzungsmittel, ein Diätetikum oder ein Arzneimittel, insbesondere im Hinblick auf Darreichungsform und Haltbarkeit, vollständig genügt. Es ist damit überraschend gelungen, erstmals ein reproduzierbares, über einen längeren Zeitraum haltbares bzw. konservierbares Stärkungsmittel auf Pastinak-Basis bereitzustellen, welches zusätzlich zu den bisher schon bekannten kräftigenden Eigenschaften des bisher als nicht reproduzierbares Naturheilmittel eingesetzten Pastinaks auch noch weitere leistungssteigernde Eigenschaften für den männlichen Organismus aufweist.

Vorzugsweise werden die Pastinak-Bestandteile aus Pastinak-Samenöl, Pastinak-Extrakten und -Auszügen und/oder getrockneten bzw. pulverisierten Pastinak-Pflanzen, insbesondere Wurzelteilen, gewonnen.

Als ganz besonders vorteilhaft hat es sich herausgestellt, wenn als Vitamine Biotin eingesetzt wird. Grundsätzlich ist Biotin an sich als ein für den Menschen essentielles Vitamin der B-Familie bekannt. Biotinabhängige Enzymkomplexe haben Schlüsselfunktionen in der Gluconeogenese. Insbesondere bei gesteigerten körperlichen Aktivitäten und Ausdauerleistungen wird über die Gluconeogenese-Reaktion den entsprechenden Muskeln und beanspruchten Organen durch den Energieträger Glucose Energie zugeführt, wodurch die Leistungsfähigkeit, Spannkraft und Ausdauer gefördert wird. Gerade bei intervallartigen körperlichen Belastungen und schubartigen Energie-konsumierenden Höchstleistungen ist die Gluconeogenese, bei der Biotin in einer Schlüsselposition sitzt, eine ganz besonders wichtige Quelle, die frische Kraft und Energie liefert. Biotin besitzt aber nicht nur bei der Gluconeogenese größte Bedeutung, sondern spielt auch eine wesentliche Rolle bei der Aufrechterhaltung der Konzentration von Citratcyclus-Zwischenprodukten. Diese Zwischenprodukte des Citratcyclus müssen mit Hilfe des Biotins immer wieder aufgefüllt werden, da sie bei der Biosynthese von Energie in Form des Energieträgers ATP oder bei der Blutsynthese ständig verbraucht werden. In Kombination mit den Pastinak-Bestandteilen entsteht somit ein ausgesprochen wirksames Stärkungsmittel.

Ganz besonders vorteilhaft ist vorgesehen, daß dieses Mittel zusätzlich auch Lecithin enthält. Lecithin ist der Sammelbegriff für eine Gruppe von Phospholipiden, bei denen die Phosphorsäure einerseits mit Cholin und andererseits mit Glycerin verestert ist, wobei die freien Hydroxylgruppen des Glycerins mit Fettsäuren wiederum verestert sind. Diese Lecithine sind die wichtigsten Ausgangssubstanzen und biologische Bausteine für Zellmembransysteme. Die wichtigsten biologischen Energieumwandlungsprozesse und Energiesyntheseprozesse laufen an zellulären Membransystemen ab, d.h. Zellmembranen sind die Produktionsstätten für den Energiestoffwechsel. Darüber hinaus besitzt Lecithin auch stabilisierende bzw. konservierende Eigenschaften, so daß ein erfindungsgemäßes Stärkungsmittel aus den wesentlichen Bestandteilen Pastinak, Biotin und Lecithin zusätzlich auch die erforderlichen Haltbarkeitseigenschaften aufweist, ohne daß es eines weiteren Stabilisators bedarf.

Diese besonders vorteilhafte erfindungsgemäße Kombination von Pastinak, Biotin und Lecithin stellt einen einzigartigen Wirkkomplex zur Stärkung insbesondere des männlichen Organismus vor allem zur Vorbeugung und Überwindung sexueller Störungen und erektiler Disfunktionen dar, da die biologischen Aktivstoffe sich nicht nur gegenseitig ergänzen, sondern darüber hinaus auch synergistisch wirken. Pastinak weckt die sexuelle Appetenz, steigert die Libido, wirkt anregend und aufbauend, fördert die Durchblutung. Lecithin fördert den natürlichen Ablauf aller erregungsbildenden sowie reizweiterleitenden Reaktionsketten und stärkt die Sensomotorik. Biotin stärkt die Leistungskraft und das Ausdauervermögen und schafft neue Energie. Refraktärzeiten werden verkürzt, daraus resultiert eine Erhöhung der Cohabitationsfrequenz.

Als besonders geeignet hat sich herausgestellt, daß das Mittel bezogen auf einen Anteil von 100 mg Pastinakbestandteilen 10 µg bis 300 µg Biotin enthält. Grundsätzlich ist es bekannt, daß in der Pastinakwurzel auch Spuren von Biotin (etwa 0,1 µg Biotin auf 100 mg Pastinakwurzel) enthalten sind, aber die vorgenannten Eigenschaften des erfindungsgemäßen Mittels werden überraschend durch die wesentlich größere Zugabe von Biotin erreicht.

Um eine besonders akzeptable Darreichungsform des erfindungsgemäßen Mittels zu erreichen, ist vorteilhaft vorgesehen, daß es kapselförmig ausgebildet ist und mittels einer Gelatinehülle ummantelt ist. Diese Gelatinehülle stellt dabei gleichzeitig auch eine zusätzliche Konservierung für die im Inneren der Hülle enthaltenden Wirkstoffe dar.

Dabei kann zusätzlich vorteilhaft vorgesehen sein, daß als Stabilisator eine ölartige Substanz eingesetzt wird.

In alternativer Ausgestaltung kann vorgesehen sein, daß das Mittel als Tonikum ausgebildet ist, wobei als Stabilisator Alkohol eingesetzt wird. Dabei ist selbstverständlich der Alkoholgehalt ausreichend niedrig einzustellen, um keine unerwünschten Nebenwirkungen bei der Einnahme des Mittels auszulösen.

Es hat sich herausgestellt, daß sich das erfindungsgemäße Mittel in der Zusammensetzung Pastinak-Biotin-Lecithin insbesondere zur Stärkung und Kräftigung des männlichen Organismus mit Libido- und Potenz-steigernden Eigenschaften eignet. Dies läßt sich durch eine Analyse der bekannten männlichen Sexualeigenschaften erklären.

Nach neueren wissenschaftlichen Erkenntnissen und bestehender Lehrbuchmeinung sind für die sexuelle Erregung des männlichen Organismus folgende physiologische Mechanismen grundlegende und unerlässliche Voraussetzung:
a) Sensorische Einflüsse
   Reize, wie zum Beispiel Aussehen, Attraktivität und Geruch des Partners gelten als sogenannte sensorische Einflüsse, die eine sexuelle Appetenz wecken, die dann eine ganze Reihe von komplexen Reflexmustern beim männlichen Organismus auslöst.
b) Motorisch-vegetative Einflüsse
   In dieser Phase erfolgt eine parasympatische und somatisch-muskulär gesteuerte Vasocongestion des männlichen Genitals. Dabei fließt sauerstoffreiches Blut rasch in die erweiterten Arterien der Muskeln und des elastischen Gewebes des männlichen Genitals, gleichzeitig wird der venöse Blutabfluß durch Kontraktion der abfließenden Gefäßabschnitte behindert. Diese Reaktion wird von den Nervenstämmen des Sakralmarks reflektorisch ausgelöst und kann zusätzlich durch descendierende Einflüsse des zentralen Nervensystems angeregt und forciert werden.

Da die Grundstrukturen der männlichen Sexualität sich in verschiedene Phasen gliedern lassen, kann jede dieser Phasen selbstverständlich auch unterschiedlichen Störeinflüssen unterliegen.
a) In den wenigsten Fällen sind erektile Disfunktionen auf organische Grundkrankheiten oder deren Begleiterscheinungen zurückzuführen. Die überwiegende Mehrzahl der erektilen Störungen sind psychologisch und psychosomatisch bedingt. Gerade hier ist ein Ansatzpunkt für die aphrodisierenden und Libido-steigernden Aktivsubstanzen des Pastinak gegeben. Das sexuelle Appetenzverhalten wird dadurch gesteigert und psychologisch bedingte Störgrößen und Hemmnisse werden hierdurch gemindert.
b) Die durch die sexuelle Appetenz ausgelösten körperlichen Reaktionen werden beim männlichen Organismus nach einem Reflexmuster nerval gesteuert. Im Mittelpunkt stehen hier komplexe Interaktionen zwischen Gehirn, sensorischen Endigungen des männlichen Genitals und afferenten sowie efferenten Nervenfasern, die an der Erregungsausbildung und Reizweiterleitung maßgeblich beteiligt sind. Alle aktiv an der Steuerung des Sexualverhaltens beteiligten nervalen Strukturen sind dabei auf die ständige de Novo-Synthese und den kontinuierlichen Einbau von Phospholipiden (Lecithinen) angewiesen, da diese Lecithine als essentieller Bestandteil von Nervenzellen für die Erregungsausbildung und Reizweiterleitung unerläßlich sind. Hier gewährleistet die vorherige Aufnahme von Lecithinen den reibungslosen Ablauf aller beschriebenen sensorischen, sensor-motorischen und motorisch vegetativen Reaktionsmechanismen und Interaktionsketten.
c) Nach erfolgreicher Vasocongestion wird in der Regel die Cohabitationsphase angestrebt. Physiologisch betrachtet zeichnet sich diese Cohabitationsphase durch rhythmische Muskelkontraktionen aus, die durch einen erhöhten Sauerstoffverbrauch und einen drastisch gesteigerten Energieaufwand gekennzeichnet sind. Der drastisch erhöhte Energieverbrauch - wie er insbesondere bei intervallartigen körperlichen Anstrengungen und Höchstleistungen eintritt - kann kurzfristig nur durch eine rasch einsetzende biochemische Gluconeogenese-Reaktion gedeckt werden, da durch die Gluconeogenese eine schnelle Neusynthese und Anreicherung von biologischen Energieträgern in der Blutbahn gewährleistet ist.
d) Kennzeichnend für die männliche Sexualdynamik sind spezifische und individuell unterschiedliche Rückbildungsphasen oder sogenannte Refraktärzeiten. Während der Refraktärzeit ist die sexuelle Ansprechbarkeit des Mannes stark reduziert. Hier versorgt die mit Hilfe Biotin-abhängiger Enzyme sprunghaft ansteigende Gluconeogenese-Reaktion den beanspruchten Organismus rasch mit neuen Energieträgern und kann somit die hinderlichen Refraktärzeiten auf ein Minimum verkürzen.

Die Erfindung ist nachstehend anhand eines Ausführungsbeispiels näher erläutert.

Zur Herstellung des Stärkungsmittels in Form einer Kapsel wird zunächst in bekannter Weise eine Kapsel aus Gelatine erzeugt, die einen inneren Hohlraum aufweist, der eine Füllmenge von etwa 350 mg aufnehmen kann. In diesen Kapselhohlraum werden folgende aktive Substanzen eingegeben:

| | |
|---|---|
| - Pastinak | 150 mg, |
| - Lecithin | 150 mg, |
| - Biotin | 50 µg. |

Der verbleibende Rest von etwa 50 mg besteht aus ölartigen Stabilisatoren.

Die vorgenannte Zusammensetzung stellt nur ein bevorzugtes Ausführungsbeispiel dar, selbstverständlich sind eine Vielzahl anderer Zusammensetzungen im Rahmen der Patentansprüche möglich.

## Patentansprüche

1. Mittel zur Stärkung des menschlichen, insbesondere des männlichen Organismus,
gekennzeichnet durch
einen Gehalt an Pastinak-Bestandteilen, Vitaminen und wenigstens einem Stabilisator.

2. Mittel nach Anspruch 1,
dadurch gekennzeichnet,
daß die Pastinak-Bestandteile aus Pastinak-Samenöl, Pastinak-Extrakten und -Auszügen und/oder getrockneten bzw. pulverisierten Pastinak-Pflanzen, insbesondere Wurzelteilen, gebildet sind.

3. Mittel nach Anspruch 1 oder 2,
dadurch gekennzeichnet,
daß als Vitamine Biotin eingesetzt wird.

4. Mittel nach Anspruch 1 oder einem der folgenden,
dadurch gekennzeichnet,
daß dieses zusätzlich Lecithin enthält.

5. Mittel nach Anspruch 1 oder einem der folgenden,
dadurch gekennzeichnet,
daß es bezogen auf einen Anteil von 100 mg Pastinakbestandteilen 10 µg bis 300 µg Biotin enthält.

6. Mittel nach Anspruch 1 oder einem der folgenden,
dadurch gekennzeichnet,
daß es kapselförmig ausgebildet ist und mittels einer Gelatinehülle ummantelt ist.

7. Mittel nach Anspruch 6,
dadurch gekennzeichnet,
daß als Stabilisator eine ölartige Substanz eingesetzt wird.

8. Mittel nach Anspruch 1, 2, 3, 4 oder 5,
dadurch gekennzeichnet,
daß es als Tonikum ausgebildet ist, wobei als Stabilisator Alkohol eingesetzt wird.
